# EUROPEAN PATENT APPLICATION

(11) **EP 3 701 880 A1**
(43) Date of publication of application: **02.09.2020**
(21) Application number: 18845464.9
(22) Date of filing: 24.10.2018
(51) Int. Cl.: A61B 10/02, A61B 10/00

(54) **A KIT FOR THE EXTRACTION OF TISSUES**

(30) Priority: 24.10.2017 AR P170102956
(71) Applicant: Odon, Jorge Ernesto, Buenos Aires (AR)
(72) Inventor: Odon, Jorge Ernesto, Buenos Aires (AR)
(74) Representative: Balder IP Law, S.L.
(86) International application number: PCT/ES2018/070694
(87) International publication number: WO 2019/081797

(57) **Abstract**

A kit for the extraction of tissues that comprises of a recipient, a sampler holder and a sampler, where the recipient comprises a socket structure with a depression or guide containing a cleaner and a lid with a depression or guide; the sampler holder has an elongated body, a handle on its proximal end and a pin on its distal end; and a sampler coupled in a removable manner to the pin of the sampler holder, where the sampler comprises a substantially cylindric body with a distal end and a proximal end, and that has a longitudinal plane in the direction of the cylindric body and a cross plane; a cutting element inside said distal end; and on its proximal end, media to couple in a removable manner the sampler to the sampler holder.

## Description

### Field of Invention

A kit for the extraction of tissues, more specifically a kit for the extraction of tissues within cavities of difficult access. Additionally, a system for the easy and secured handling of the extracted tissue is provided.

### State of the Art

US patent 5,827,199 of Melvin Alexander discloses an apparatus to extract samples that comprises a hollow arm, with an open end. A circular shaped projection acts as support for the fingers that operate the apparatus. The open end has a circular cutting blade.

US patent 8,187,292 of Khalid AIGhamdi discloses an apparatus to remove samples of skin. It comprises an elongated arm with a partially circular cutting element, or having a "C" shape on one of its ends.

US patent 8,337,414 of James Vetter et al., discloses a device to remove tissues, made up of a circular element that is inserted into the tissue. The circular element is made up of two parallel hollow cylinders, with a gap in between. Within said gap, a flexible blade is provided. To separate the sample from the rest of the tissue, a flexible blade is operated that initially slides lineally and, after contacting with a stopper on the inner side of the external cylinder, it deviates towards the centre of the circular element.

### Objectives

An objective of the present invention is to provide a kit for the extraction of tissues, especially within cavities of difficult access.

An objective of the present invention is to provide a kit for the extraction of tonsils.

Another objective of the present invention is to provide a kit for the extraction of tissues of easy handling.

Another objective of the present invention is to provide a kit for the extraction of tissues where the operator does not have any contact with said tissue.

Another objective of the present invention is to provide a kit for the extraction of tissues where the tissue is kept in a sterile recipient.

Another objective of the present invention is to provide a kit for the extraction of tissues where simultaneously with the extraction of tissue it cauterizes the wound to prevent bleeding.

### Figures

Figure 1 shows the components of the kit for the extraction of tissues.
Figure 2 shows a sampler in a blade configuration.
Figure 3 shows a sampler in a helix configuration.
Figure 4 shows a sampler in a cutting wire configuration.
Figure 5 shows a specific sampler for the extraction of tonsils.
Figure 6 shows the configuration of the kit to cauterize.
Figure 7 shows the socket structure located in the lower part of the recipient.
Figure 8 shows the deposit lid of the sample.
Figure 9 shows a longitudinal cut of the cap.

### Summary of the invention

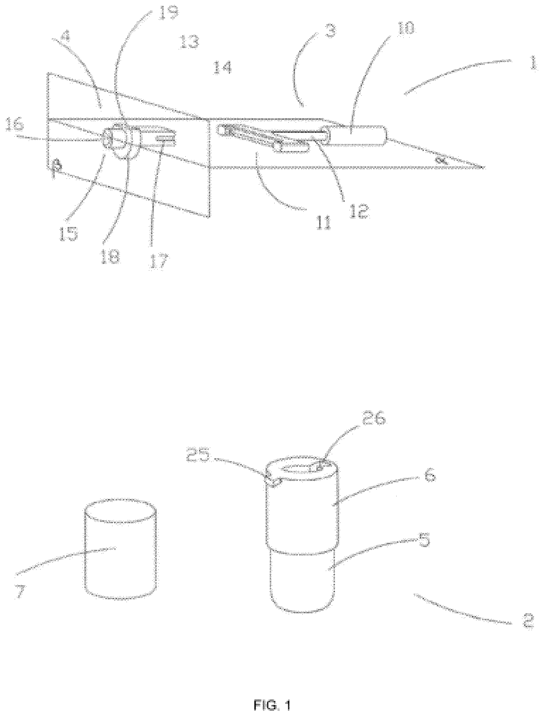

Figure 1 shows the kit 1 for the extraction of tissues of the present invention. Said kit is comprised of a deposit of extracted tissue 2, a sampler holder 3 and a sampler 4.

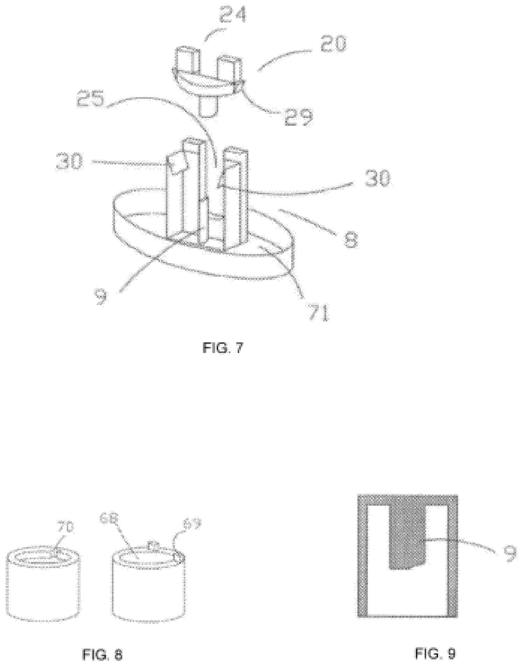

The deposit of the extracted tissue comprises a recipient 5 with its corresponding lid 6 and a cap 7 and a socket structure 8 (Figure 7) provided in the bottom of the recipient or on the bottom of the cap, in which the sampler is fixed and which is comprised of a cleaner 9 (Figure 7) to sweep the tissue adhered to the sampler.

The sampler holder comprises a handle 10 on its proximal end, a coupling medium 11 on its distal end and a body 12 between both ends. The depth of access to the tissue to be extracted shall determine the length of the body. The coupling medium on its distal end, which is coupled, in a removable manner, to said sampler. It may provide an electrical connection with the sampler.

The sampler is a body 13 substantially cylindric, it is the element that is in contact with the tissue, and the one to exert the cutting action for its extraction. It has a longitudinal plane (a) in direction of the cylindric body and that passes through its centre, in the direction in which thrust forces are applied, and a cross plane (β), perpendicular to the longitudinal, for the rotatory movements. It presents a proximal end 14 and a distal end 15. The body is a hollow cylinder, having on its distal end an edge that may be sharp to facilitate the insertion, in longitudinal direction, of the sampler into the tissue. In the interior of the hollow cylinder, a cutting and retaining element 16 is provided, having a structure and made of a material that, when applying a thrust and rotatory movement, is capable of cutting and separating the tissue, which will be kept within the sampler body.

The sampler provides, on its proximal end, a coupling medium 17, and it couples, in a removable manner, to the coupling medium on the distal end of the sampler holder, having a configuration of a removable coupling between both components.

The sampler has a distal end adapted to be inserted into the tissue by thrust and that, by means of a rotatory or of a thrust and rotatory movement, cuts and separates a portion of tissue to be extracted. The sampler has a stopper 18, to determine the depth of collecting the sample. This stopper may present a chamfer 19 to allow the passage of light and to observe the operation of extracting the tissue. This stopper may be fixed, adjustable or supplemental to vary the depth of the cut. In case of being adjustable, the depth may be regulated by means of a threaded joint between the sampler and the stopper, or be supplemented with washers of different thickness. In this last case, washers may have a socket to fix them to the sampler and/or a chamfer. The stopper has, as well, a cleaner (not shown) to clean the sampler of the tissue adhered to its surfaces.

The external side of the sampler has at least one lock that will prevent the removal of the sampler from the socket structure, once it has been socketed, so that its re-use is avoided, providing same the characteristic of being disposable.

### Detailed description of the invention

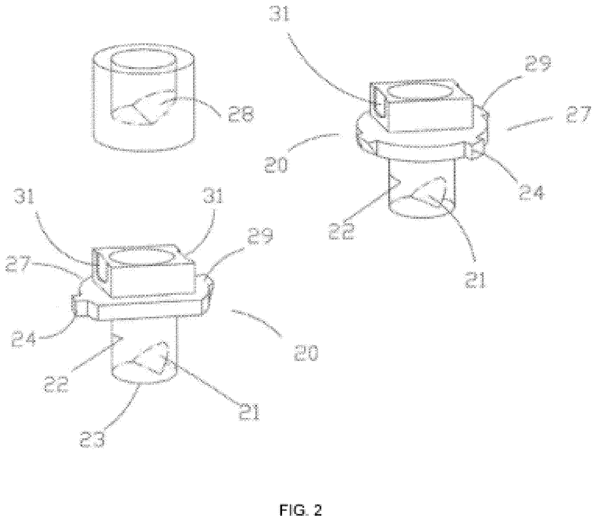

Figure 2 shows a first embodiment of the sampler. In this, the sampler is a hollow cylinder 20, where an inclined blade 21 is developed in its inner part, and is fixed in a portion of the inner surface 22 of the hollow cylinder. Said blade has a configuration and a material able to cut the tissue, and it substantially extends transversally from the periphery of the hollow cylinder up to its centre or slightly forward, so that when rotating it cuts a slice of tissue to be extracted apart from the rest of the tissue. The configuration of the blade is such that, when it rotates together with the sampler, it cuts the tissue in a helicoidal manner, being able to extract the cut tissue together with the sampler. The sampler has a distal end that presents an edge 23. In one configuration, said edge may be sharp to facilitate the cut of the tissue to be extracted in longitudinal direction. In one configuration, the cylinder is longitudinally separated in two halves, the latter being able to move close to or apart from each other. This relative movement between both halves of the hollow cylinder may be parallel or inclined, where the distal end, the one of the edge, presents a different diameter than the one of the proximal end. This configuration allows the area of the tissue to be extracted to be regulated, instead of changing the sampler for one of different size.

Optionally, on the outer side of the sampler, at least one guide 24 is provided which will fit in stoppers 25 or depressions 26 in the deposit lid of the extracted tissue, or on the socket structure, so that the sampler has a unique socket position within the deposit of extracted tissue. On the outer side of the body of the sampler, a stopper 27, fixed or variable, may be provided, to determine the depth at which the tissue is extracted.

The external side of the sampler has at least one lock 28 that will prevent the removal of the sampler from the socket structure, once it has been socketed, so that its re-use is avoided, providing same the characteristic of being disposable.

In the present configuration, the cleaner 29 is a plug, appropriate for a provided socket structure (not shown), either in the bottom of the cap or in the bottom of the recipient. The sampler with the tissue contained in its interior is fitted into the socket structure, in one unique position. The plug tightly fits into the inner diameter of the sampler and sweeps said tissue as long as the sampler is inserted into the socket structure until it gets fixed by means of locks 30 that prevent its removal. Once this position is reached, the sampler holder is removed, preferably with a movement of inclination to uncouple it from the sampler. In the version in which the socket structure is fixed to the bottom of the recipient, the same socket movement will make the plug sweep the tissue within the hollow cylinder. In the version in which the socket structure is found in the bottom of the cap, first the sampler is fixed to the orifice on the recipient lid, then the cap is inserted to cover the recipient, in a unique possible position along the guides, where the cleaner sweeps the tissue adhered to the inner surface of the sampler.

In the coupling configuration, in a preferred configuration, there are two cavities 31, diametrically opposed in the proximal end of the sampler, into which the pins of the distal end of the sampler holder shall tightly and removably fit.

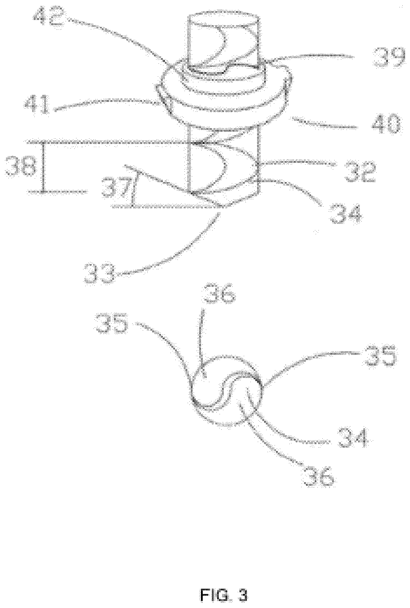

Figure 3 shows a second embodiment of the sampler. In this, the sampler is a drill bit 32, collinear with the sampler. It has a reduced dead centre 33 and at least one helix 34 with a sharp leading edge 35. Said at least one helix has a substantially curved section 36 of small thickness, so that said section has substantially reduced occupation of material, thus obtaining a greater space to gather the tissue. The peak angle 37 and the pitch (half pitch 38 in the figure) of helix shall be sufficient for the drill bit be inserted into the tissue and be sunk a predetermined depth.

When used, the drill bit is pressed against the tissue so that when rotating the sampler, same is inserted into the tissue at a determined depth, cutting the tissue. Then the sampler is removed, dragging the tissue to be extracted.

In a preferred configuration, the proximal end of the sampler provides a depression 39 through which the helix of the drill bit passes. In this configuration, the cleaner is said depression provided on the proximal end of the sampler. After socketing the sampler into the socket structure, either in the bottom of the recipient or in the bottom of the cap, the drill bit is removed by rotating same in the opposite direction, so that said movement of the helix with respect to the depression sweeps the tissue adhered to the surfaces of said helix and to the inner edges of the sampler.

On the external side of the body of the sampler, a stopper 40, fixed or variable, may be provided to determine the depth at which the tissue is extracted. The external side of the sampler has at least one lock 41 that will prevent the removal of the sampler out of the socket structure, once it has been socketed, so that its re-use is avoided, providing same the characteristic of being disposable.

In other configuration, the proximal end of the sampler has a washer 42 that may rotate around the longitudinal axis and that is provided with a depression along which the helix passes. After socketing the sampler into the socket structure, either in the bottom of the recipient or in the bottom of the cap, the drill bit is removed without rotating in proximal direction, so that the washer rotates around its longitudinal axis by following the design of the helix, sweeping tissue adhered to the surfaces of said helix and to the inner edges of the sampler.

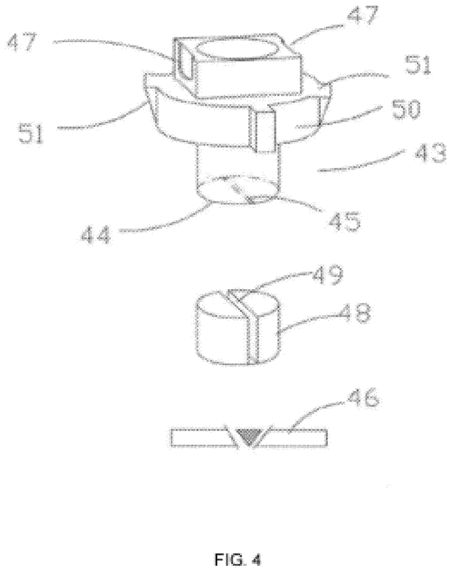

Figure 4 shows a third embodiment of the sampler. In this, the sampler is a hollow cylindric piece 43 and has, at its distal end, a circumferential edge 44 and a cutting wire 45. The cutting wire may be a resistant wire element that passes, in a cross plane, through the centre of the cylindric hollow body. The cutting wire may also have a triangular cross-sectional piece 46 with its sharp vertexes, so that said piece presents a sharp edge that may be capable of cutting the tissue on its three vertexes. That is, it may cut the tissue on the longitudinal and cross planes. Said circumferential edge may be sharp. When used, the tissue is pressed so that the circumferential edge together with the cutting wire is inserted into the tissue at a determined depth. Then the sampler is rotated so that the cutting wire transversally cuts the portion of tissue inside the cylindric piece. When the sampler is removed, same drags the tissue.

In the coupling configuration, in a preferred configuration, there are two cavities 47, diametrically opposed in the proximal end of the sampler, into which the pins of the distal end of the sampler holder shall tightly and removably fit.

In the present configuration, the cleaner 48 is a circular plug with a longitudinal cut 49, appropriate for a provided socket structure, either in the bottom of the cap or in the bottom of the recipient. The sampler with tissue contained in its interior is fitted within the socket structure, in a unique position. The plug tightly fits within the inner diameter of the sampler and the cutting wire into the longitudinal cut, and sweeps said tissue as long as the sampler is inserted into the socket structure until it gets fixed by means of locks that prevent its removal. Once this position is reached, the sampler holder is removed, preferably with a movement of inclination to uncouple it from the sampler. In the version in which the socket structure is fixed to the bottom of the recipient, the same socket movement will make the plug sweep the tissue within the hollow cylinder. In the version in which the socket structure is found in the bottom of the cap, first the sampler is fixed to the orifice on the recipient lid, then the cap is inserted to cover the recipient, in a unique possible position along the guides, where the cleaner sweeps the tissue adhered to the inner surface of the sampler.

On the external side of the body of the sampler, a stopper 50, fixed or variable, may be provided to determine the depth at which the tissue is extracted. The external side of the sampler has at least one lock 51 that will prevent the removal of the sampler out of the socket structure, once it has been socketed, so that its re-use is avoided, providing same the characteristic of being disposable.

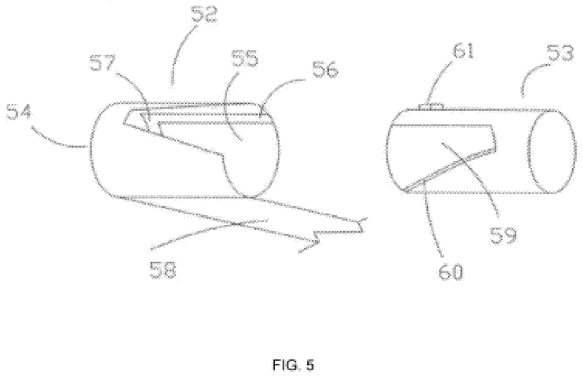

Figure 5 shows a fourth embodiment of the sampler, specifically for the extraction of tonsils. In this, the sampler is made up of two pieces, a lid of the sampler 52 and the body of the sampler 53. The lid of the sampler has a covered cylinder 54 at its distal end and, at its proximal periphery, a first "U" shaped cut 55. On the inner side, a longitudinal slot 56 is provided followed by a cross slot 57. The lid of the sampler has a support 58. The body of the sampler has at its distal end a second "U" shaped cut 59. On the inner side, and on the side of the second "U" shaped cut, a flat blade 60 is provided. The blade may be inclined with respect to the cylinder axis. The body of the sampler has a lateral guide 61 that may slide along the longitudinal slot up to the bottom, and then rotate within the cross depression.

The configuration is such that when the body of the guide is inserted into the lid of the guide, both "U" shaped cuts coincide, leaving a hollow space and, when both pieces rotate together, the flat blade advances over the first "U" shaped cut.

In its specific use to extract tonsils, the first "U" shaped cut of the sampler lid is placed behind the tonsil and the capsule inside the first "U" shaped cut. Then, the body of the sampler is inserted into the sampler lid, so that, along the guides and depressions, the second "U" shaped cut coincides with the first "U" shaped cut, keeping the tonsil enclosed in the sampler lid and the capsule in said hollow space. The sampler body is rotated following the cross slot so that the flat blade cuts, preferably in an inclined manner, the capsule to fully separate same from the pharynx. Once the complete cut of the capsule is attained, the instruments are removed together with the tonsil in their interior.

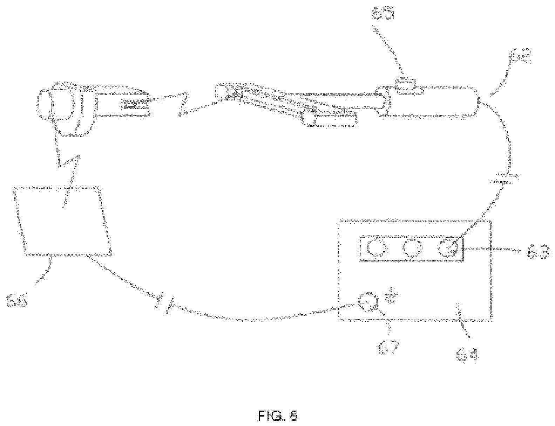

Figure 6 shows a variation of the embodiments previously described, where the kit for the extraction of tissues may be prepared for the cauterization at the time of extracting the tissue, preventing the bleeding of the wound made when cutting the tissue. In this variation, the cutting element must be of metal, electrically connected to the coupling medium of the sampler holder, along which the electric circuit continues up to the handle. The coupling media between the sampler holder and the sampler must be electrically and removably connected. The handle has a connection 62 to any of the monopolar outlets 63 of a cauterization equipment (electrocautery) 64. The handle may include a push-button 65 to close the electric circuit for the cauterization.

In its use for the simultaneous extraction of tissue and cauterization, a return plate 66 is provided, connected to the grounding 67 of the electrocautery. In this way, when closing the circuit with the push-button, or another key in any other place of the circuit, the electric circuit that will heat the cutting element, causing the cauterization of the tissue, is closed.

The sampler holder is a substantially elongated piece and is able to transmit both the thrust force of the sampler into the tissue and the rotation of the assembly making a cross cut. Its length is defined based on the depth and ease of access to the tissue from where the sample is to be taken. It may provide a chamfer for the passage of light towards the tissue within the cavity.

The proximal end of the sampler holder has a handle to operate the sampler holder. It may be a widening of the section of the body or it may be a paddle that allows holding it tightly to be pressed against the tissue, and that transmits the rotation to the sampler on the opposite end. They preferably have rough surfaces to favor their operation.

The central section of the sampler holder may be circular, have a cross shape or have the shape of another section that will transmit the forces that intervene in the taking of the sample. In the case that the determination of the rotation angle is required, a reference is provided on the sampler holder.

The distal end of the sampler holder ends in a pitchfork with two arms that fit into the cavities of the proximal end of the sampler that completes the coupling configuration with the sampler. The tight fit, for example bayonet or depression-projection, allows transmitting the pressure and the rotation of the sampler. A simple movement of inclination of the body with respect to the sampler shall be sufficient to separate both pieces. For the cauterization configuration, both the arms of the pitchfork and the cavities in which they fit have metallic surfaces to close the electric circuit.

The deposit of tissue is a recipient, conveniently sterile and prepared to accommodate the size of the tissue sample, with a lid and a cap.

Figure 7 shows a deposit lid of the extracted tissue. It has an orifice 68 in which the sampler is tightly socketed. To place the sampler on the lid in a unique position, there is at least one guide 69 or depression 70 on the lid in which the at least one guide of the sampler is fitted. Once socketed, the sampler holder is uncoupled, so that this only keeps fixed to the lid. By being socketed on the lid, the sampler together with the sample taken remain inside the recipient without having had any contact with the operator.

Figure 8 shows a socket structure, that fixes the sampler to the recipient, and that contains the cleaner that cleans the tissue adhered inside the sampler. In one embodiment, said socket structure is located in the inner part of the cap, which surrounds an upper portion of the body of the recipient. It has a cleaner that, when placing the sampler into the socket structure, said cleaner sweeps the tissue adhered to the surfaces of the sampler inside the deposit.

In another embodiment, said socket structure is located in the bottom of the recipient 71. It has a cleaner that, when placing the sampler into the socket structure, said cleaner sweeps the tissue adhered to the surfaces of the sampler inside the deposit.

Preferably, media are provided so that the sampler is socketed in one unique position with respect to the socket structure.

To assure that the kit is disposable, at least two security flanges 56 are provided between the socket structure and the sampler, so that once locked, they can only be uncoupled by means of breaking some element.

## Claims

1. A kit for the extraction of tissues, **CHARACTERIZED in that** it comprises a recipient, a sampler holder and a sampler, where:
the recipient comprises a socket structure with a depression or guide containing a cleaner and a lid with a depression or guide;
the sampler holder has an elongated body, a handle on its proximal end and a pin on its distal end; and
a sampler coupled in a removable manner to the pin of the sampler holder, where the sampler comprises:
a substantially cylindric body with a distal end and a proximal end, and that has a longitudinal plane in the direction of the cylindric body and a cross plane;
a cutting element inside said distal end; and
on its proximal end, media to couple in a removable manner the sampler to the sampler holder.

2. The kit for the extraction of tissues according to claim 1, **CHARACTERIZED in that** the distal end of said substantially cylindric body has a sharp edge.

3. The kit for the extraction of tissues according to claim 1, **CHARACTERIZED in that** the proximal end of said substantially cylindric body has at least one stopper that fits in said depression or guide of the orifice of the lid or of the socket structure.

4. The kit for the extraction of tissues according to claim 1, **CHARACTERIZED in that** the sampler has an outer stopper, fixed or variable, to determine the depth at which the tissue is extracted.

5. The kit for the extraction of tissues according to claim 4, **CHARACTERIZED in that** the variable outer stopper is regulated by means of a threaded joint o with supplements that are socketed to the sampler.

6. The kit for the extraction of tissues according to claim 1, **CHARACTERIZED in that** the cutting element comprises an inclined blade, fixed in at least one portion of the inner surface of the hollow cylinder, and that extends from the edge up to the centre of the cylinder or slightly forward.

7. The kit for the extraction of tissues according to claim 1, **CHARACTERIZED in that** the cutting element is a drill bit with at least one helix with a sharp edge, and the proximal part of the sampler provides a depression that may copy the helix and its passage.

8. The kit for the extraction of tissues according to claim 1, **CHARACTERIZED in that** the cutting element is a cutting wire, a wire type element, in the cross plane, that passes through the centre of the cylindric body with a sharp edge capable of cutting the tissue.

9. The kit for the extraction of tissues according to claim 8, **CHARACTERIZED in that** the cutting wire is a triangular cross-sectional piece that passes through the centre of the cylinder body, with its ends fixed to the edge of the cylindric body, and where its three vertexes are sharp.

10. The kit for the extraction of tissues according to claim 1, **CHARACTERIZED in that** it is adapted for cauterization, having an electric circuit that circulates through the sampler holder, through the coupling media, through the sampler and even through the metal cutting element, completing the circuit with a return plate and electrocautery.

11. The kit for the extraction of tissues according to claim 1, **CHARACTERIZED in that** it provides locking flanges that are operated when placing the sampler inside the socket structure and that prevent the removal of the sampler from said socket structure.

12. The kit for the extraction of tissues according to claim 1, **CHARACTERIZED in that** the socket structure is provided on the inner side of the cap, in which case the cap is provided with an orifice with at least one depression or guide and is apt to receive and fix the sampler, so that when placing the cap on the lid, the cleaner of said socket structure tightly fits inside the hollow cylinder of the sampler.

13. The kit for the extraction of tissues according to claim 1, **CHARACTERIZED in that** the socket structure is provided in the bottom of the recipient, so that when placing the sampler into the socket structure, the cleaner of said socket structure tightly fits inside the hollow cylinder of the sampler.

14. The kit for the extraction of tissues according to claim 1, **CHARACTERIZED in that** the sampler is made up of a sampler lid and a sampler body, where the sampler lid has a covered cylinder at its distal end and, at its proximal periphery, a first "U" shaped cut, a longitudinal slot on the inner side followed by a cross slot, a support of the proximal side with a slot that follows the longitudinal slot, and where the sampler body has on its distal end a second "U" shaped cut, and on the inner side, and on the side of said second "U" shaped cut a flat blade is provided, and where the sampler body has a lateral guide that may slide along the longitudinal slot to the bottom, and then rotate inside the cross depression.
